# EUROPÄISCHE PATENTANMELDUNG

(11) **EP 2 017 756 A1**
(43) Veröffentlichungstag der Anmeldung: **21.01.2009**
(21) Anmeldenummer: 07014276.5
(22) Anmeldetag: 20.07.2007
(51) Int. Cl.: G06F 19/00

(54) **Verfahren zur Anzeige und/oder Bearbeitung bzw. Verarbeitung von Bilddaten medizinischen oder medizintechnischen Ursprungs mit Gestenerkennung**

(71) Anmelder: BrainLAB AG, 85622 Feldkirchen (DE)
(72) Erfinder: Steinle, Wolfgang, 80337 München (DE); Frielinghaus, Nils, 85551 Heimstetten (DE); Hamilton, Christoffer, 81371 München (DE); Gschwandtner, Michael, 80469 München (DE)
(74) Vertreter: Schwabe - Sandmair - Marx

(57) **Zusammenfassung**

Die Erfindung betrifft ein Verfahren zur Anzeige und/oder Bearbeitung bzw. Verarbeitung von Bilddaten medizinischen oder medizintechnischen Ursprungs, unter Verwendung eines Anzeigegeräts (1) mit mindestens einem Bildschirm (2, 3), wobei
- die Bilddaten durch eine im Anzeigegerät (1) integrierte Computer-Datenverarbeitungseinheit (4) verarbeitet werden, um Bildausgaben (14) zu erzeugen und/oder die Bilddaten zu ändern bzw. zu bestätigen,
- die Bilddaten durch Eingaben am Bildschirm (2, 3) selbst manipuliert, erzeugt oder aufgerufen werden, und wobei
- die Eingaben mit Hilfe der Datenverarbeitungseinheit (4) über eine Gestenerkennung identifiziert werden, wobei die Gesten per Hand oder mit Gestenerzeugungsgeräten erzeugt werden.

## Beschreibung

Die Erfindung betrifft ein Verfahren zur Anzeige und/oder Bearbeitung bzw. Verarbeitung von Bilddaten medizinischen oder medizintechnischen Ursprungs.

Medizinisches oder medizintechnisches Bildmaterial, das mit Hilfe medizintechnischer Bildgebungsverfahren erzeugt wird, wie z.B. mit Computertomographie, Kernspintomographie oder Röntgengeräten (zweidimensional/dreidimensional) wird in immer größerem Umfang als digitales Bildmaterial bzw. als digitaler Bilddatensatz gespeichert. Hierfür verwendete Systeme tragen die englische Bezeichnung "Picture Archiving and Communication System (PACS)". Die primäre Betrachtung bzw. Auswertung solcher digitalen Bilder beschränkt sich derzeit auf Radiologen, die in dafür vorgesehenen Betrachtungsräumen mit hochauflösenden Monitoren hoher Leuchtkraft arbeiten, wodurch in Krankenhäusern Einsparungen bezüglich des verwendeten Filmmaterials erzielt werden können.

Außerhalb der Radiologie schreitet der Übergang zur filmlosen Bildbetrachtung langsamer fort. Es werden beispielsweise Bilder, die in der Radiologie filmlos betrachtet werden, auf Film ausgedruckt, um einer sekundären Verwendung in anderen Abteilungen zugänglich gemacht zu werden. Dies mag einerseits daran liegen, dass PACS-Computerprogramme sehr auf Radiologen abgestimmt sind; andererseits ist ihre Bedienung oftmals kompliziert. Hinzu kommt noch, dass viele Ärzte daran gewöhnt sind, mit einem von hinten beleuchteten Schaukasten zu arbeiten, der auch "Lightbox" genannt wird.

Es sind Anstrengungen unternommen worden, digitales Bildmaterial für die sekundäre Verwendung außerhalb der Radiologie besser zugänglich zu machen, beispielsweise durch Großbildmonitore in Operationssälen, die durch kabellose Tastaturen oder Mäuse bedient werden können. Auch einfache Touchscreen-Bedienungen werden verwendet, oder es werden separate Kameras bereitgestellt, welche Steuerungseingaben von Ärzten oder Bedienungspersonal erkennen können. Ein Anzeigesystem für medizinische Bilder, das in der Art eines Schaukastens bzw. einer Lightbox aufgebaut ist, ist aus der US-2002/0039084 A1 bekannt. Hier werden verschiedene Möglichkeiten zur Manipulation der medizinischen Bilder angegeben, beispielsweise Eingaben über ein separates Kontrollfeld, Fernbedienungen, einfache Touchscreen-Applikationen oder Sprachsteuerung.

Es ist die Aufgabe der vorliegenden Erfindung, die Betrachtung und Manipulation von Bildern bzw. Bilddaten medizinischen oder medizintechnischen Ursprungs, beispielsweise Patientenbilddaten, einfach und intuitiv zu gestalten und insbesondere auch in Operationssälen bzw. für die sekundäre Verwendung nach der Radiologie in optimierter Weise zugänglich zu machen.

Diese Aufgabe wird erfindungsgemäß durch ein Verfahren gemäß dem Anspruch 1 gelöst. Die Unteransprüche definieren bevorzugte Ausführungsformen der Erfindung.

Bei dem erfindungsgemäßen Verfahren zur Anzeige und/oder Bearbeitung bzw. Verarbeitung von Bilddaten medizinischen oder medizintechnischen Ursprungs wird ein Anzeigegerät mit mindestens einem Bildschirm verwendet, wobei
- die Bilddaten durch eine im Anzeigegerät integrierte Computer-Datenverarbeitungseinheit verarbeitet werden, um Bildausgaben zu erzeugen und/oder die Bilddaten zu ändern bzw. zu bestätigen,
- die Bilddaten durch Eingaben am Bildschirm selbst manipuliert, erzeugt oder aufgerufen werden, und wobei
- die Eingaben mit Hilfe der Datenverarbeitungseinheit über eine Gestenerkennung identifiziert werden, wobei die Gesten per Hand oder mit Gestenerzeugungsgeräten erzeugt werden.

Anders ausgedrückt stellt die Erfindung eine digitale Lightbox mit einem optimierten Befehlseingabesystem zur Verfügung, das auf der Verarbeitung von durch einen Benutzer ausgeführten Gesten beruht, die unmittelbar am Bildschirm ausgeführt werden können oder durch eine Erfassung detektiert werden, die unmittelbar dem Bildschirm zugeordnet ist. Solche Gesten sind im Sinne der vorliegenden Erfindung Eingaben, denen ihrer Natur nach eine spezielle Bedeutung zukommt, oder denen vom Anzeigegerät oder seinen Komponenten eine spezielle Bedeutung zugeordnet werden kann.

Eine solche Gestenerkennung zusammen mit der Eingabe am Bildschirm durch dem Bildschirm zugeordnete Eingabe-Erkennungsmittel gestattet dem Benutzer eine schnelle und intuitive Bildbetrachtung, sie macht Bildbetrachtungssysteme geeignet für Operationssäle, insbesondere weil die notwendige Sterilität aufrechterhalten werden kann. Bildbetrachtungssysteme, die das erfindungsgemäße Verfahren verwenden, können in der Art von Schaukästen bzw. Lightboxen wandmontiert bereitgestellt werden und so dem Benutzer sein gewohntes Arbeitsumfeld bereitstellen. Komplizierte und aufwändige bzw. schwer zu sterilisierende Bedienungsmittel wie Mäuse und Tastaturen oder Eingabetastenfelder müssen nicht mehr bereitgestellt oder bedient werden, wobei darüber hinaus die Gestenerkennung eine Möglichkeit zur vielfältigeren Betrachtung und Bildmanipulation bietet als dies bei herkömmlichen Systemen generell der Fall war.

Die Erfindung wird nunmehr anhand mehrerer Ausführungsformen näher erläutert. Sie kann alle hierin beschriebenen Merkmale einzeln sowie in jedweder sinnvollen Kombination umfassen: In den beiliegenden Zeichnungen zeigen:
- Figur 1: ein schematisches Abbild der erfindungsgemäßen digitalen Lightbox;
- Figur 2: eine Darstellung einer flächenartigen Eingabe;
- Figuren 3a bis 3d: Bildbetrachtungsbeispiele;
- Figuren 4a bis 4c: ein Beispiel für eine Bildschirmausschnitt-Vergrößerung;
- Figuren 5a bis 5d: ein Beispiel für die Erzeugung eines Polygonzuges;
- Figuren 6a bis 6d: Beispiele für das Spiegeln bzw. Kippen eines Bildes;
- Figuren 7a und 7b: Beispiele für das Aufrufen eines versteckten Menüs;
- Figuren 8a bis 8c: Beispiele für eine Bildschirmtastaturbedienung;
- Figuren 9a bis 9d: Beispiele für eine Scroll-Bedienung;
- Figuren 10a bis 10c: ein Beispiel für eine Punktanwahl in einem Diagramm;
- Figuren 11a bis 11f: Beispiele für Diagrammmanipulationen;
- Figur 12: ein Beispiel für eine Linkshänder/Rechtshänder-Erkennung;
- Figuren 13a bis 13c: Beispiele für eine Linienerzeugung bzw. -manipulation;
- Figuren 14a bis 14h: Beispiele für die Manipulation von Bilddarstellungen für Patientendatensätze;
- Figuren 15a bis 15d: Beispiele für Punktzuordnungen;
- Figur 16: ein Beispiel für eine Befehlsbestätigung;
- Figur 17: ein Beispiel für eine Objektvermessung;
- Figuren 18a und 18b: Beispiele für eine Kreiskontur-Erzeugung;
- Figur 19: ein Beispiel für die Manipulation eines Implantats;
- Figuren 20a bis 20c: ein Beispiel für eine bildinhaltsabhängige Eingabeinterpretation;
- Figur 21: ein Beispiel für eine Countdown-Einstellung;
- Figur 22: ein Beispiel für eine Signatureingabe; und
- Figuren 23a bis 23c: Beispiele für die Manipulation mehrerer Bildelemente.

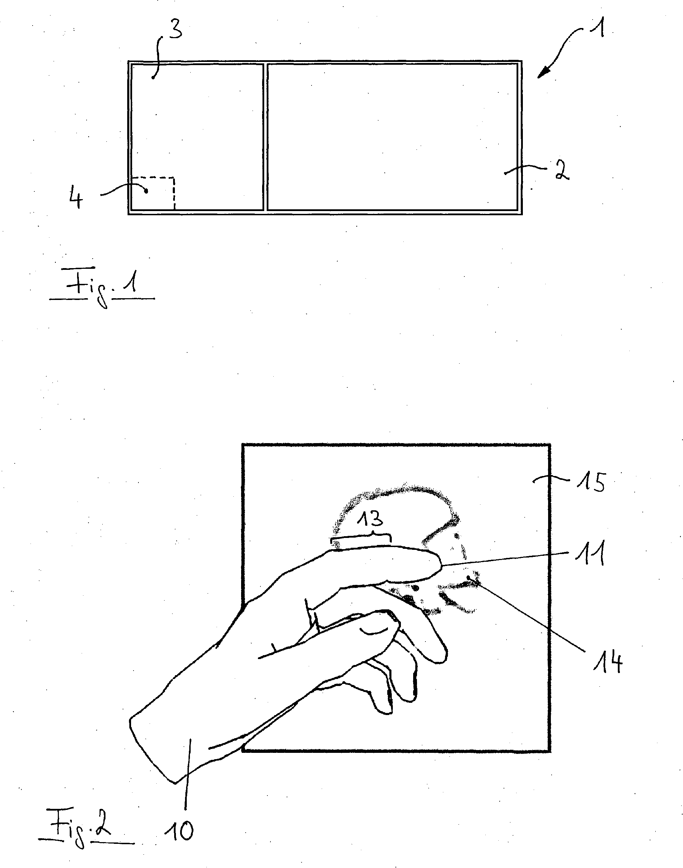

Die Figur 1 zeigt in schematischer Darstellung eine digitale Lightbox, mit der das Verfahren gemäß der vorliegenden Erfindung umgesetzt werden kann. Die digitale Lightbox (Anzeigegerät) 1 umfasst zwei separate Bildschirme oder Bildschirmteile 2, 3 und eine integrierte Computer-Datenverarbeitungseinheit 4, die nur schematisch dargestellt ist. Über die Computer-Datenverarbeitungseinheit 4 können einerseits Bilddatensätze in die Lightbox 1 geladen werden, andererseits wird durch sie die Darstellung der Bilddatensätze gesteuert, und zwar gemäß der Eingabegesten, die im weiteren noch anhand vieler Beispiele besprochen werden. Natürlich kann die Datenverarbeitungseinheit 4 optional auch Änderungen oder Ergänzungen feststellen, die durch die Eingaben vorgenommen werden und kann die Datensätze entsprechend verändern. Die Bildschirme bzw. Bildschirmteile 2, 3 sind bei diesem erfindungsgemäßen Beispiel als sogenannte Multitouch-Bildschirme ausgebildet. Bei dieser Technik können mehrere Eingaben gleichzeitig erfasst werden, beispielsweise Eingaben an verschiedenen Bildschirmstellen oder flächenhafte Eingaben. Der Bildschirm kann Eingaben durch tatsächliche Berührungen der Bildschirmoberfläche erfassen oder Präsenzen in der Nähe der Bildschirmoberfläche, was beispielsweise durch den Aufbau eines Infrarotstrahlengitters ermöglicht wird.

Durch den integralen Einbau der Datenverarbeitungseinheit 4 entsteht eine abgeschlossene Einheit, die - ebenso wie ein Lichtkasten - an einer Wand befestigt werden kann. Die beiden Bildschirme bzw. Monitore 2 und 3 sind nebeneinander angeordnet, wobei der kleinere Monitor 3 bei einer bevorzugten Ausführungsform eine Steuerungsschnittstelle (beispielsweise für den Datentransfer, die Eingabebefehls-Zuordnung oder die Auswahl von Bildern bzw. Bilddaten) bereitstellt und auf dem größeren Monitor die Bilder selbst dargestellt werden. Die Breite des kleineren Monitors 3 entspricht der Höhe des größeren Monitors 2, und der kleinere Monitor 3 ist um 90 Grad gedreht, wodurch eine große Bedienfläche entsteht.

Anhand der Figur 2 soll erläutert werden, wie im Rahmen der vorliegenden Erfindung flächenhafte Eingabegesten erzeugt werden können. Die Figur 2 zeigt einen Bildschirmausschnitt 15, auf dem ein Bild 14 angezeigt wird, hier ein schematisches Patientenkopf-Abbild. Es ist die Hand 10 eines Bedieners zu sehen, wobei am linken Zeigefinger der Bereich des zweiten Fingergliedes als flächenhafter Bereich mit dem Bezugszeichen 13 und die Spitze des Zeigefingers als Punkt 11 angezeigt sind. Im Rahmen der vorliegenden Erfindung kann nun eine Bedienungsperson beispielsweise eine flächenhafte Berührung des Bildschirms mit dem Zeigefingerbereich 13 (oder auch mit dem gesamten Finger) durchführen. Andererseits kann die Berührung beispielsweise nur mit der Fingerspitze 11 erfolgen.

Wenn hier und im weiteren der Begriff "Berührung" für eine Eingabe am Bildschirm gewählt wird, so soll dieser Begriff zumindest die beiden oben schon angesprochenen Eingabearten am Bildschirm umfassen, nämlich einerseits eine tatsächliche Berührung des Bildschirm und andererseits die Erzeugung einer Präsenz an der Bildschirmoberfläche bzw. in einer (moderaten) Entfernung von der Bildschirmoberfläche. Wie in der Figur 2 dargestellt, kann also der Bediener im Rahmen der vorliegenden Erfindung unterschiedliche Eingabegesten durchführen, die einerseits punktförmige Berührungen und andererseits flächenhafte Berührungen umfassen können. Solche unterschiedlichen Eingaben können auch unterschiedlich interpretiert werden und dieser Umstand stattet den Bediener mit einer weiteren Dimension für die Dateneingabe aus. Einige Beispiele für unterschiedliche Eingabeinterpretationen, die einer flächenhaften Berührung oder einer punktförmigen Berührung zugeordnet können und sich durch die Berührungsarten unterscheiden lassen, sind das Verschieben von Bildern auf dem Bildschirm, das Auswählen einer Position in einer Scrollleiste bei einer anderen, ähnlichen Benutzerschnittstelle; die Bewegung einer Scrollbar-Zeigers auf eine gewählte Position zur schnelleren Auswahl in einem Scrollfeld; das Abspielen oder Pausieren animierter Bildsequenzen oder auch eine Optionsauswahl in einem Feld mit mehreren (scrollbaren) Optionen, beispielsweise durch das Ändern der Sortierungsart. Auf einiger dieser Möglichkeiten und auf weitere Möglichkeiten wird später noch detaillierter Bezug genommen.

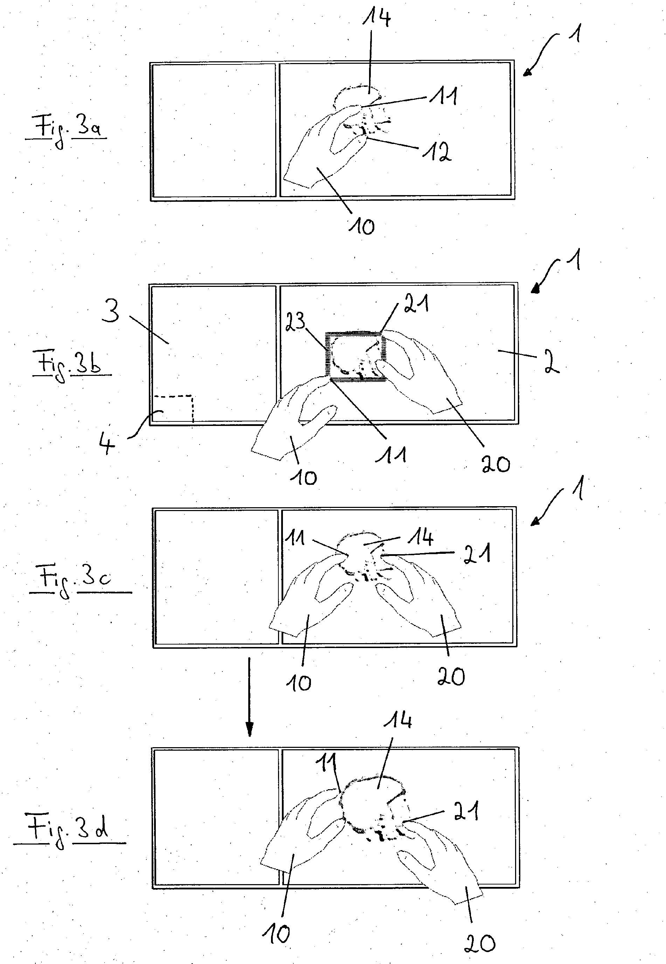

Die Figuren 3a bis 3d zeigen Einsatzmöglichkeiten der vorliegenden Erfindung bei der Bildbetrachtung. Die Figur 3a zeigt beispielsweise, wie durch eine Berührung mit einer oder zwei Fingerspitzen 11, 12 einer Hand, ein ausgewähltes Bild 14 beeinflusst werden kann, und ein Beispiel für eine solche Beeinflussung könnte das Modifizieren der Helligkeit und des Kontrastes durch ein Zusammenspiel von Gesten sein, die mit der bzw. den Fingerspitzen 11, 12 durchgeführt werden. Die Helligkeit kann beispielsweise dadurch verstellt werden, dass eine einzige Fingerspitze den Bildschirm berührt und dann eine horizontale Bewegung durchgeführt wird, während eine vertikale Bewegung den Kontrast verstellt. Ein weiteres Beispiel wäre ein Auseinander- und Zusammenbringen der Fingerspitzen 11, 12, wenn das Programm so eingestellt ist, dass es entsprechend auf solche Gesten reagiert.

Die Figur 3b zeigt, wie ein gewisser Bildschirmausschnitt mit Hilfe von zwei Fingerspitzen 11, 21 zweier Hände 10, 20 angewählt werden kann, was durch die rechteckige Umrisslinie 23 optisch dargestellt wird. Mit dem so hergestellten Bildausschnitt kann dann entsprechend den Wünschen des Betrachters durch geeignete Eingaben weiter verfahren werden. Der Umriss 23 kann beispielsweise durch eine gleichzeitige Berührung mit den beiden Fingerspitzen 11, 21 erzeugt werden. In den Figuren 3c und 3d ist dargestellt, wie eine Geste des gleichzeitigen Berührens eines Bildes mit den Fingerspitzen 11, 21 und dann des Auseinanderziehens dieser Fingerspitzen dazu führt, dass das Bild 14 vergrößert wird. Es ist anzumerken, dass entsprechende Befehlszuordnungen in der Gestenerkennungs-Software der Computer-Datenverarbeitungseinheit 4 hinterlegt und zugeordnet werden können, und dass es außerdem möglich ist, solche Zuordnungen zu ändern, beispielsweise durch die vorherige Auswahl einer bestimmten Interpretation am linken, kleinen Bildschirm 3 der Lightbox 1. Dies kann grundsätzlich für alle weiteren oder vorherigen Ausführungsbeispiele ebenso gelten.

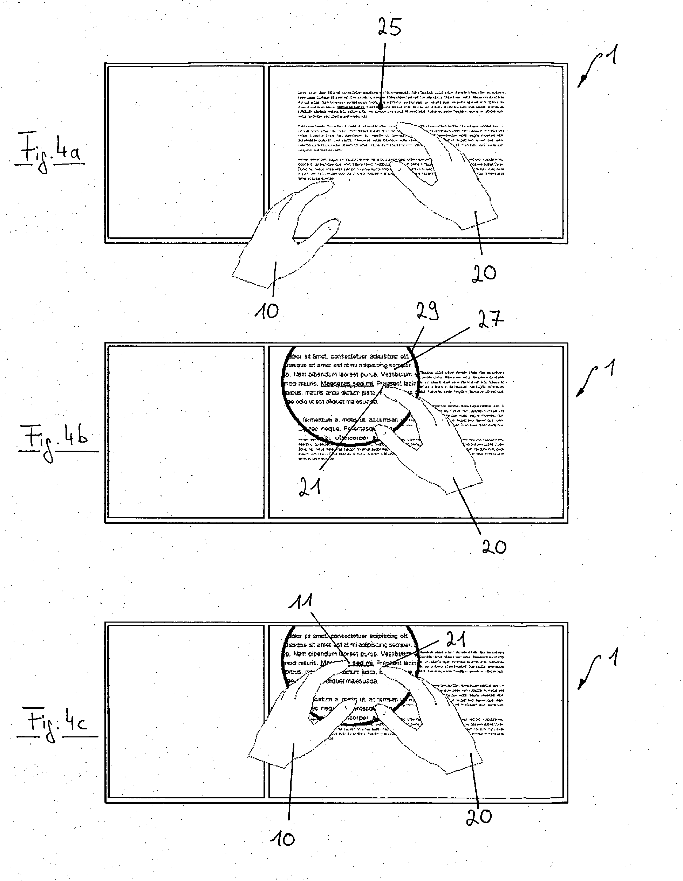

Ein Vergrößerungs-Merkmal gemäß der vorliegenden Erfindung kann anhand der Figuren 4a bis 4c erläutert werden. Wenn beispielsweise ein Text 25 auf dem Bildschirm dargestellt wird, kann die Gestenerkennung eine Zuordnung umfassen, bei welcher eine erste Bildschirmberührung mit der Fingerspitze 21 einen Bereich in der Umgebung des Berührungspunktes vergrößert, der dann in der Art einer Bildschirmlupe mit dem Rand 29 dargestellt wird. Der Text 27 ist in diesem Bereich vergrößert dargestellt, und es besteht die Möglichkeit - siehe Figur 4c - mit einer zweiten Berührung parallel oder nachfolgend (und dann gleichzeitig) zur ersten Berührung eine Selektion im Text vorzunehmen, beispielsweise die Auswahl eines Hyperlinks, wenn die zweite Berührung innerhalb des vergrößerten Bereichs stattfindet. Mit der zweiten Berührung kann aber alternativ auch ein anderer Vorgang ausgelöst werden, beispielsweise das Markieren einer Bildstelle, die nicht unbedingt ein Textbaustein sein muss, sondern auch ein bestimmter Teil in einer anatomischen Darstellung sein kann.

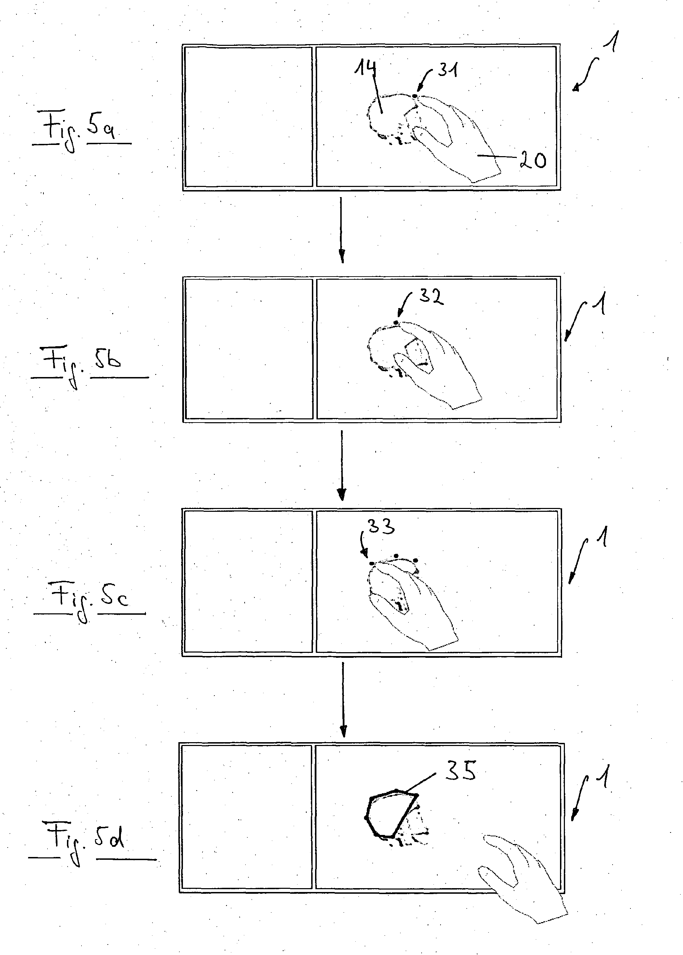

Eine Ausführungsvariante, bei der mit Hilfe eines erfindungsgemäßen Verfahrens ein Polygonzug erzeugt wird, ist in den Figuren 5a bis 5d zu sehen. Hier löst eine Folge von Berührungen die Auswahl bzw. Definition eines interessierenden Bereichs aus, beispielsweise einer Knochenstruktur in einem medizinischen Bild 14. Die erste Berührung 31 wird als Startpunkt für den interessierenden Bereich bzw. Polygonzug interpretiert, und solange der erste Punkt 31 aktiv bleibt (dazu kann, muss aber nicht unbedingt eine Fingerspitze auf dem ersten Punkt verbleiben) werden danach folgende Berührungen als weitere Punkte auf der Begrenzungslinie des interessierenden Bereiches interpretiert. Durch eine Rückkehr zum ersten Punkt über weitere Punkte 32, 33 usw. kann indiziert werden, dass der Bereich vollständig definiert ist; dies kann aber auch durch eine andere Berührungsfolge oder durch das Entfernen aller Berührungen geschehen. Am Ende ist dann der Bereich, der von Interesse ist, mit dem Polygonzug 35 markiert (Figur 5d).

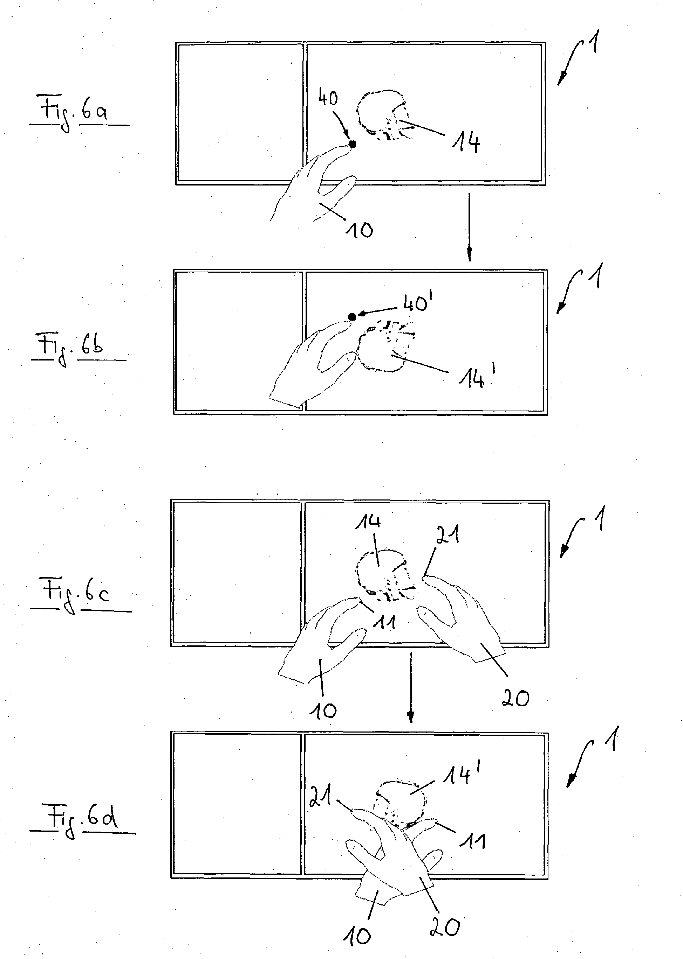

Eine weitere Bildmanipulation wird anhand der Figuren 6a bis 6d aufgezeigt, nämlich das Spiegeln bzw. das Kippen eines Bildes 14 auf der Lightbox 1. Die beiden Figuren 6a und 6b zeigen, wie ein Bild 14 um eine horizontale Achse gekippt bzw. gespiegelt werden kann, indem ein virtueller Knopf 40, der separat für diese Geste bereitgestellt wird, mittels einer Fingerspitze von unten nach oben horizontal gekippt wird. Wenn die Verschiebung in horizontaler Richtung erfolgt, kann eine entsprechende Verkippung um eine vertikale Achse stattfinden. Nach dem Durchführen des Kippvorgangs verbleibt der Knopf an der verschobenen Stelle 40', so dass er auch als Anzeige dafür dienen kann, dass das Bild gekippt bzw. gespiegelt worden ist.

Eine zweihändige Kipp- bzw. Spiegelungsgeste ist in den Figuren 6c und 6d aufgezeigt. Wenn die beiden Fingerspitzen 11 und 21 der Hände 10 und 20 bei einer Bildberührung aufeinander zu und aneinander vorbei geschoben werden, wird dies bei dieser Ausführungsform als ein Befehl interpretiert, das Bild 14 um eine vertikale Achse zu kippen bzw. zu spiegeln. Natürlich ist durch eine entsprechende, gegenläufige Fingerbewegung in vertikaler Richtung auch ein Spiegeln um eine horizontale Achse möglich.

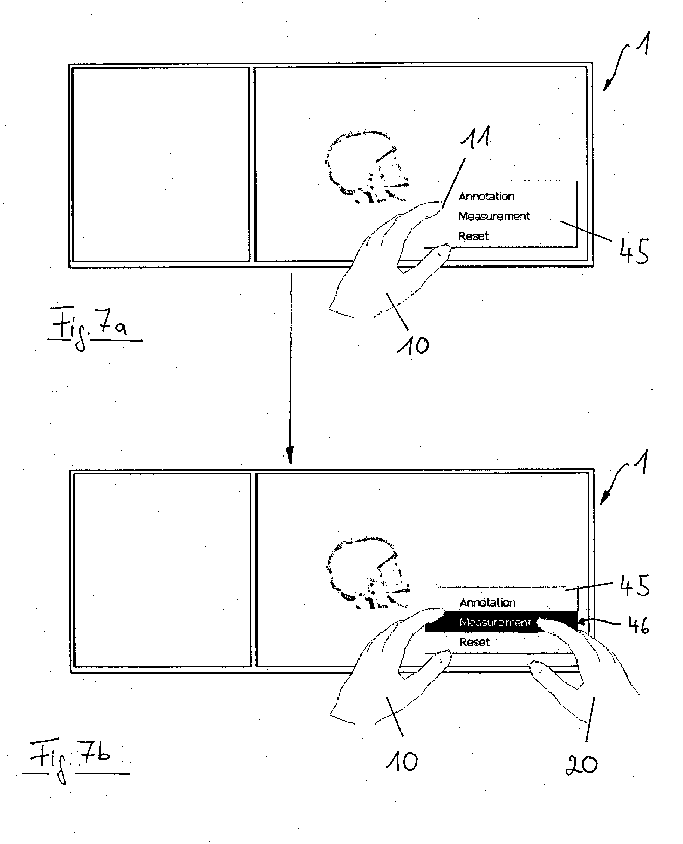

Die in den Figuren 7a und 7b dargestellte Eingabe betrifft das Aufrufen eines ansonsten versteckten Menüfeldes 45 durch eine erste Fingerspitzenberührung 11 (Figur 7a), worauf dann mit einer zweiten Berührung eine Auswahl in dem aufgeklappten Menü erfolgen kann, beispielsweise hier die Auswahl des mittleren Befehlsfeldes 46.

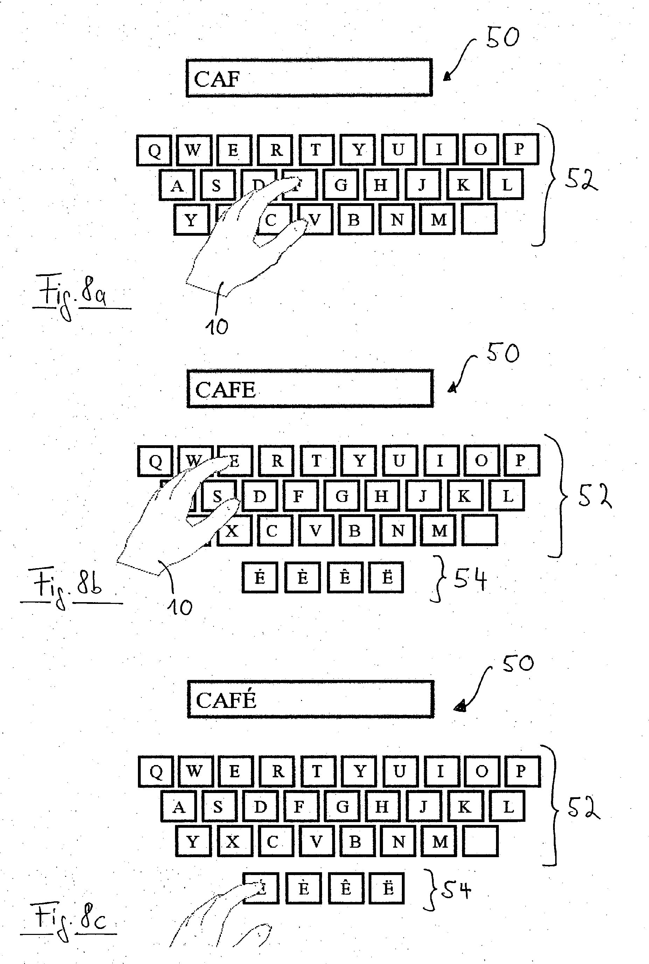

Die Ausführungsvariante gemäß den Figuren 8a bis 8c betrifft die Eingabe von Zeichen über eine Bildschirmtastatur. Es können mehr Tasteneingaben getätigt werden als bei den üblichen Tastaturen mit 101 Tasten, beispielsweise kann die Eingabe aller 191 Zeichen gemäß IS08859-1 unterstützt werden, und zwar dadurch, dass einer virtuellen Taste mehrere Zeichen zugeordnet werden. Die Zeichen werden unter Verwendung von Ähnlichkeitskriterien zugeordnet, beispielsweise sind dem Zeichen E mehrere andere E-Zeichen mit unterschiedlichen Akzenten zugeordnet. Nachdem das Zeichen E auf dem Tastaturabschnitt 52 ausgewählt wird, werden in einem zusätzlichen Tastaturabschnitt 54 verschiedene alternative Zeichen angeboten (Figur 8b), während das Zeichen E in seiner Grundform schon in die Kontrollausgabe 50 geschrieben wird. Wenn dann, wie in Figur 8c gezeigt, ein Sonderzeichen E mit einem Akzent aus der Reihe 54 ausgewählt wird, wird das zuletzt eingegebene Zeichen durch dieses Sonderzeichen ersetzt.

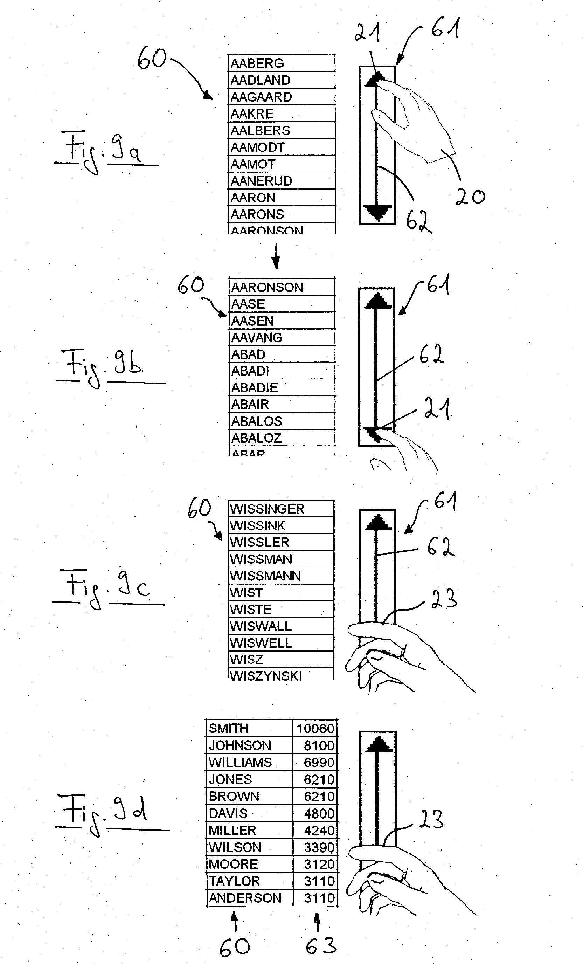

Eine Scrollbar-Bedienung bzw. -Auswahl unter Verwendung der vorliegenden Erfindung wird anhand der Figuren 9a bis 9d erläutert. Mit dem Bezugszeichen 60 ist in diesen Figuren eine alphabetische Namensliste gezeigt, die durch eine Scrollbar 61 durchgeblättert bzw. von oben nach unten und umgekehrt verschoben werden kann. Dazu umfasst die Scrollbar 61 einen Scrollpfeil bzw. Scrollbereich 62. In Figur 9d ist noch die Liste 60 erweitert, nämlich um eine Ziffernkolonne 63. Ein Scrollen durch die Liste 60 kann erfindungsgemäß durch die Berührung der Scrollbar 61 im Pfeilbereich 62 erfolgen, und indem die Fingerspitze 21 nach unten geführt wird, wird nach unten in der Liste 60 weitergeblättert, wie den Figuren 9a und 9b zu entnehmen ist. Ein Ziehen der Fingerspitze 21 mit Berührung am Bildschirm führt zu diesem Vorgang. Man kann aber auch ein Element oder einen bestimmten Bereich auswählen, indem man, wie in Figur 9c gezeigt, mit dem zweiten Zeigerfingerglied 23 eine flächenmäßige Berührung auf der Scrollbar 61 durchführt. Wenn durch eine solche flächenmäßige Berührung eine bestimmte Stelle am Pfeil 62 berührt wird, springt die Liste zu einer entsprechenden relativen Position und der ausgewählte Bereich wird angezeigt. Bei einer weiteren Ausführungsform kann durch eine andere als eine punktförmige Auswahl auch die Anzeige- oder Scroll-Reihenfolge geändert werden. Hier ist beispielsweise in Figur 9d gezeigt, dass eine flächenmäßige Berührung mit dem zweiten Fingerglied 23 dazu führt, dass eine zweite Liste 63 geöffnet wird, auf der durch ein Auf- und Abbewegen des Fingers gescrollt werden kann.

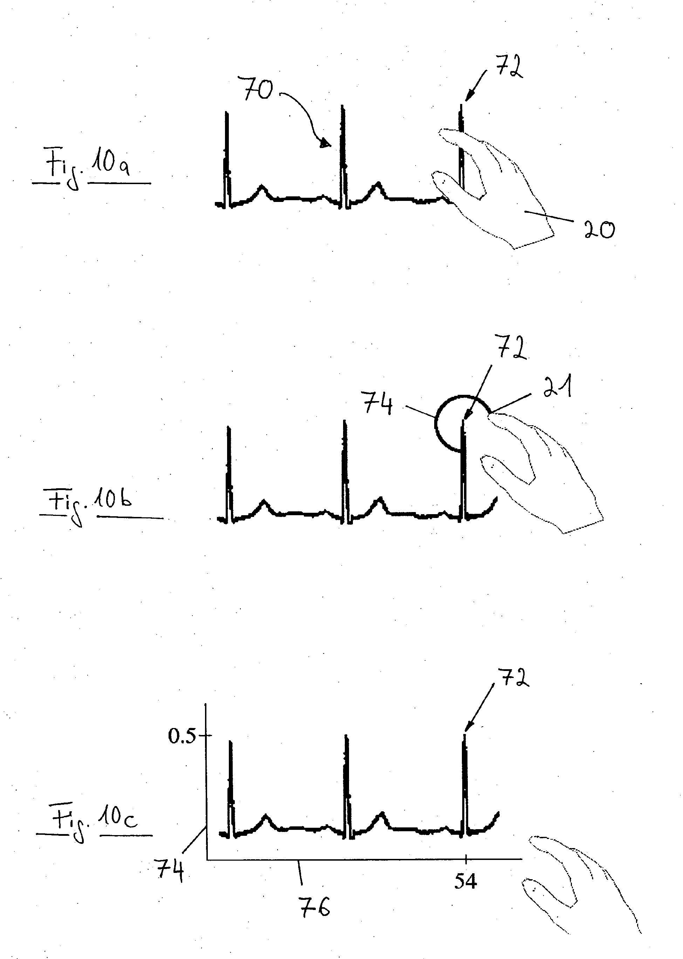

Eine Erfindungsvariante, bei der Diagramme manipuliert werden, ist in den Figuren 10a bis 10c gezeigt. Das Diagramm 70, hier ein EKG eines Patienten weist beispielsweise die Spitze 72 auf (Figur 10a). Will nun ein Verwender Genaueres über den Wert an dieser Spitze 72 erfahren, kann er - wie in Figur 10b gezeigt - den Punkt 72 durch das Einkreisen mit seiner Fingerspitze 21 auswählen, worauf zur Bestätigung beispielsweise der Auswahlkreis 74 erscheint. Auf diese Auswahl hin kann die Computer-Datenverarbeitungseinheit nunmehr auf den Diagrammachsen 74, 76 die Werte ausgeben, welche die Spitze 72 betreffen, hier 0,5 auf der Achse 74 und 54 auf der Achse 76. Ähnliche Auswertungen sind für andere Messungen oder beispielsweise für Eigenschaften wie Farbwerte des ausgewählten Punktes oder einer ausgewählten Fläche möglich.

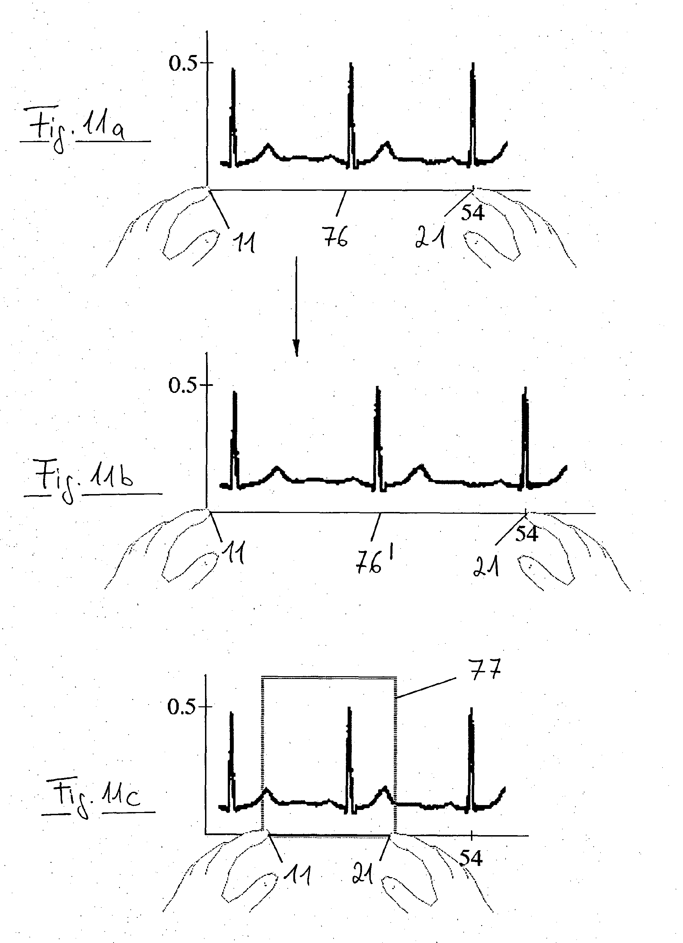

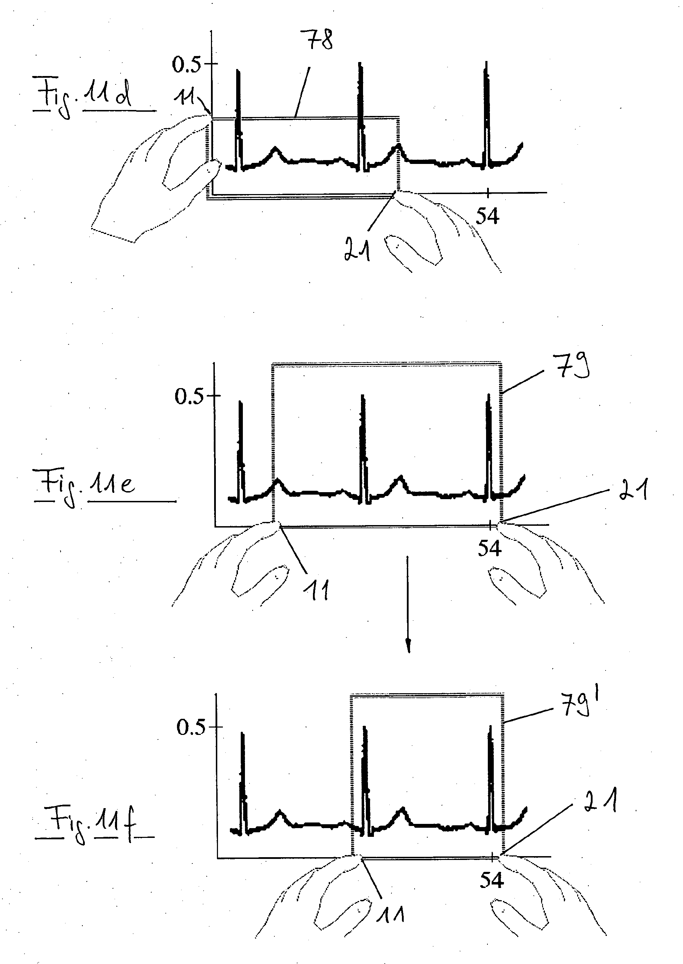

Auch die Manipulation von Diagrammen ist als Ausführungsbeispiel der Erfindung möglich. Ein Diagramm kann beispielsweise, wie in den Figuren 11a und 11 b gezeigt, durch zwei Fingerspitzenberührungen skaliert werden, wenn beispielsweise die Fingerspitze 11 den Ursprung berührt und dort verbleibt und die Fingerspitze 21 einen Punkt auf der Achse 76 nach rechts verschiebt, so dass eine breiter skalierte Achse 76' entsteht. Die Figuren 11 c und 11 d zeigen zwei verschiedene Möglichkeiten, einen Diagrammbereich auszuwählen. In Figur 11c wird der Diagrammbereich durch zwei Fingerspitzenberührungen 11, 21 auf der unteren Achse gewählt und die Höhe des Auswahlbereiches 77 wird automatisch so bestimmt, dass sie die wichtigen Diagrammteile umfasst. Eine Auswahl bei der die Höhe selbst gewählt wird, ist für den Bereich 78 in Figur 11d zu sehen, wobei die Fingerspitzenberührungen 11 und 21 gegenüberliegende Ecken eines Rechtecksbereiches 78 definieren. Natürlich können auch schon vorhandene Auswahlen neu eingestellt werden, wie z.B. der Auswahlbereich 79 (Figur 11e), der durch ein Verschieben der Fingerspitze 11 in den Bereich 79' geändert wird.

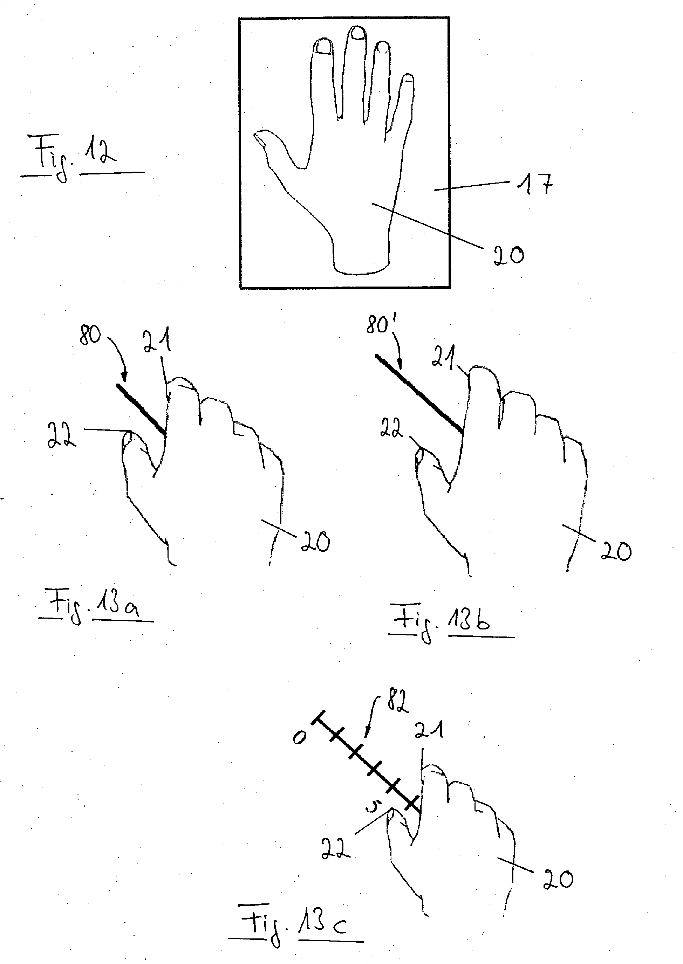

Die Figur 12 zeigt, wie unter Verwendung der vorliegenden Erfindung einer Lightbox bzw. deren Datenverarbeitungseinheit mitgeteilt werden kann, ob der Verwender Rechts- oder Linkshänder ist. Ein flächenhaftes Auflegen der Hand 20 auf einen Bildschirmbereich 17 bewirkt mehrere Berührungen, und durch eine Erfassung der Größe unterschiedlicher Berührungspunkte sowie der Abstände zwischen den Berührungen kann - beispielsweise durch einen Modellvergleich - ermittelt werden, ob es sich um eine rechte oder eine linke Hand handelt. Hiernach kann die Benutzerschnittstelle bzw. die Anzeige entsprechend so eingestellt werden, dass sie für den jeweiligen Typ bequem und optimal handhabbar ist. Dass es sich hierbei um eine solche Feststellung handelt, kann von der Datenverarbeitungseinheit bei einer Ausführungsform beispielsweise dann festgestellt werden, wenn eine Handauflage über eine gewisse Zeit erfolgt.

Es ist grundsätzlich auch möglich, mit Hilfe des erfindungsgemäßen Verfahrens das Bildmaterial bzw. die Bilddatensätze zu ergänzen, insbesondere Objekte oder Hilfslinien einzuzeichnen. Die Figuren 13a bis 13c zeigen Beispiele hierfür. In einem hierfür eingestellten Modus kann der Benutzer zwei Fingerspitzen 21, 22 mit dem Bildschirm in Berührung bringen, und durch diese Geste wird eine Linie 80 gezeichnet. Wenn der Verwender nun - wie in Figur 13b - die Fingerspitzen 21, 22 weiter auseinander nimmt, wird die rechtwinklig zur Fingerspitzenverbindung definierte Linie gestreckt, die Linienlänge wird also relativ zum Fingerspitzenabstand definiert. In gleicher Weise kann in einem weiteren Modus, wie in Figur 13c dargestellt, ein Lineal 82 erzeugt werden, dessen Skalierung vom Abstand der Fingerspitzen 21, 22 abhängt.

Bei diesem Beispiel wird auch deutlich, dass bei der vorliegenden Erfindung ganz allgemein die Bedeutung der Eingabegesten von einem vorher zu wählenden oder sich selbst durch die Gesten ergebenden bzw. aus der Geste identifizierbaren Eingabemodus abhängen kann.

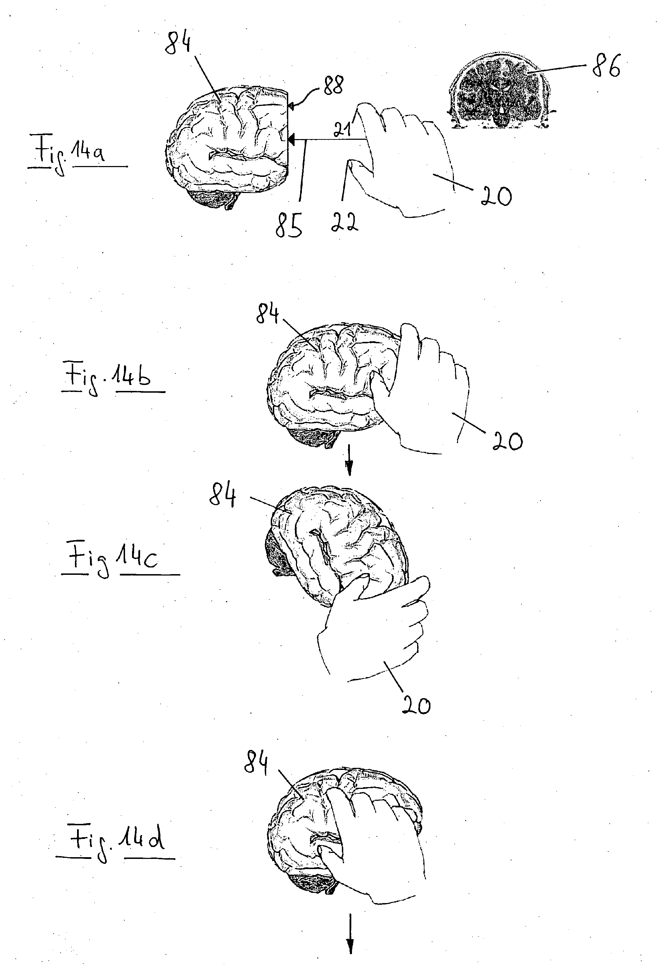

Zwei- und dreidimensionale Bildmanipulationen, wie sie mit Hilfe der vorliegenden Erfindung ausführbar sind, sind beispielhaft in den Figuren 14a bis 14h aufgezeigt. Durch die Verwendung multipler Berührungen kann ein Objekt manipuliert werden, das als dreidimensionales Modell oder dreidimensionale Rekonstruktion eines Patientenscans auf dem Bildschirm angezeigt wird.

So ist in Figur 14a gezeigt wie eine Schnittebene an einem Gehirn 84 bestimmt und angezeigt werden kann. Die Schnittebene 88 ist eine Ebene, auf die der Pfeil 85 zeigt. Der Pfeil 85 wird durch zwei Fingerspitzenberührungen 21, 22 erzeugt, und seine Länge hängt von dem Abstand der Fingerspitzen 21, 22 ab. Er führt senkrecht auf die Ebene 88. Werden nun die Fingerspitzen 21, 22 weiter auseinander bewegt oder näher zueinander gebracht, ändert sich die Lage der Schnittebene 88, und ein entsprechendes Schnittbild kann nebenan zusätzlich dargestellt werden, wie mit dem Bezugszeichen 86 angedeutet ist. Durch das Bewegen der Fingerspitzen 21, 22 kann also die Darstellung 86 als orthogonale Schnittebene durch verschiedene Schnittebenen hindurch "gescrollt" werden.

Die Figuren 14b und 14c zeigen, wie durch die Verschiebung zweier Berührungen in einer Drehbewegung ein dreidimensionales Objekt um eine Achse rotiert wird, die parallel zur Blickrichtung liegt und auf der Linie zwischen den beiden Berührungen zentriert wird.

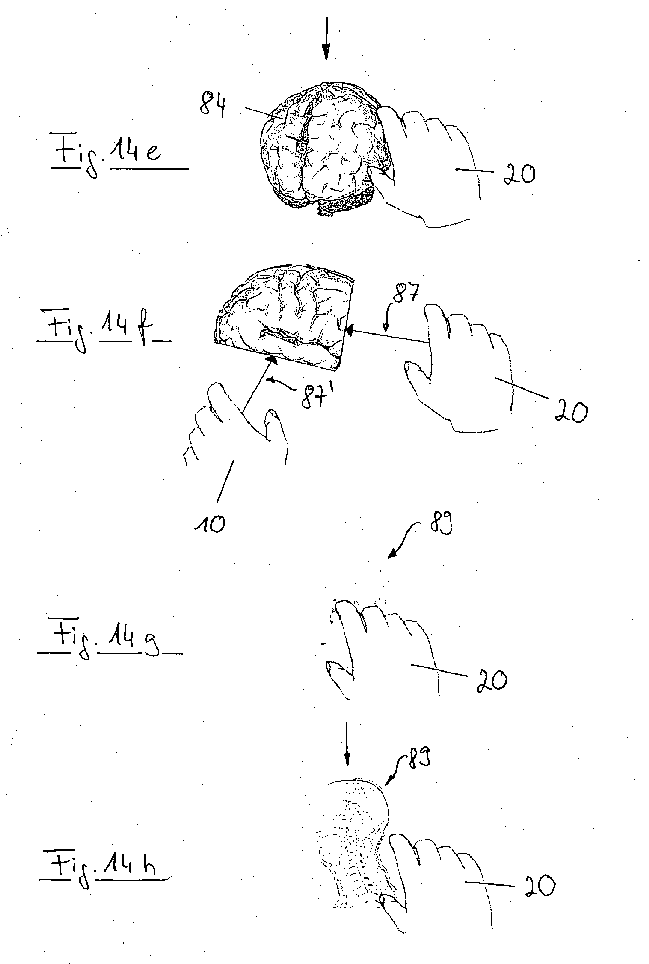

Wenn, wie in den Figuren 14d und 14e dargestellt ist, zwei Berührungen in derselben Richtung verschoben bzw. gezogen werden, wird das 3D-Objekt 84 um eine Achse gedreht, die senkrecht zur Blickrichtung liegt, und zwar parallel zur Linie zwischen den beiden Punkten und zentriert auf der Mitte des 3D-Objekts. Die Figur 14f zeigt, wie zwei Zwei-Finger-Linien 87, 87' verwendet werden können, um ähnlich wie bei Figur 14a Schnittebenen zu erzeugen, wobei ein dreidimensionaler Objekt-Keil definiert werden kann.

Die Figuren 14g und 14h zeigen schließlich, dass die beschriebenen Dreh- bzw. Rotationsvorgänge auch auf zweidimensionale Darstellungen angewendet werden können, die aus einem dreidimensionalen Datensatz stammen oder einander in sonstiger Weise zugeordnet worden sind. Mit einer Bewegung einer Zwei-Finger-Berührung parallel zu einer Seite hin, wird die Darstellung 89 um 90 Grad vom Zustand der Figur 14g zu dem Zustand der Figur 14h gedreht. In solcher Weise können Datensatzausrichtungen zwischen sagittal, axial und koronar verändert werden. Wenn beispielsweise ein sagittales Bild vorliegt, würde eine Positionierung der Fingerberührungen auf dem Oberteil des Bildes und ein Ziehen der Berührung nach unten die Ausrichtung zu einer axialen Ausrichtung verhindern.

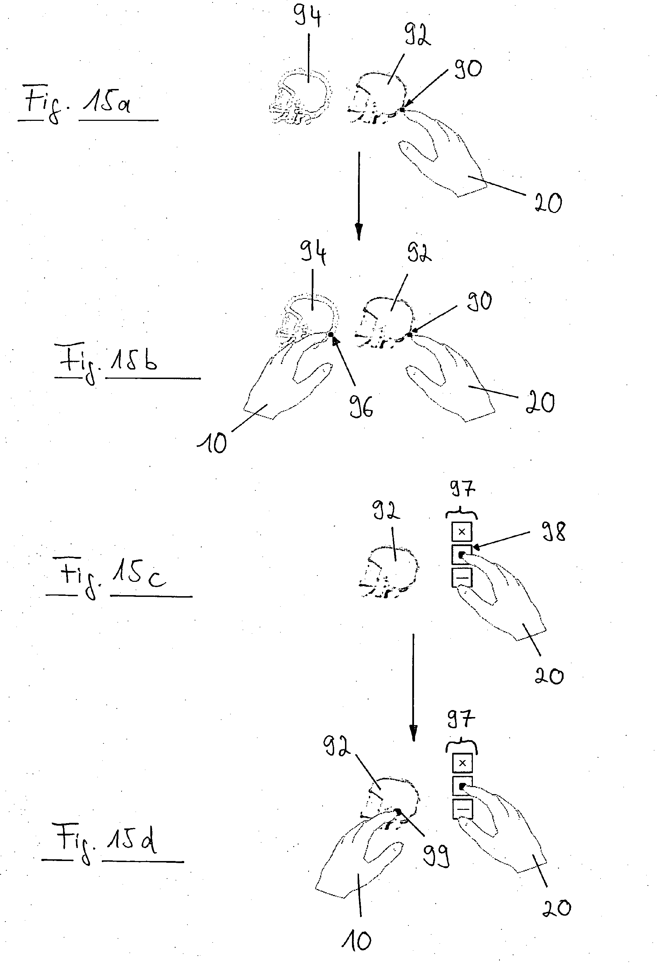

Ein weiteres Anwendungsfeld der Erfindung betrifft das sogenannte "Pairing", d.h. das Zuordnen zweier oder mehrerer Objektpunkte. Beispielsweise bei der Registrierung beim Fusionieren oder beim Matching von zwei verschiedenen Bildern können Einzelpunkte aus beiden Bildern als derselbe Objektpunkt identifiziert und zugeordnet werden, und so zeigen die Figuren 15a und 15b, wie auf den Bildern 92 und 94 zunächst der Punkt 90 mit einer Fingerspitze markiert wird und dann der entsprechende Punkt 96 auf dem anderen Bild. So genannte Image-Fusion-Techniken können diese Randbedingungen hilfsweise verwenden. Die Figuren 15c und 15d zeigen eine weitere Ausführungsform, bei der zunächst ein GUI-Element (Graphic User Interface-Element) 98 aus einer Auswahl 97 durch eine Fingerspitzenberührung gewählt wird, um ein Label auszuwählen, wonach die Fingerspitzenberührung mit der anderen Hand 10 dann das Label 99 an der gewünschten Stelle anbringt.

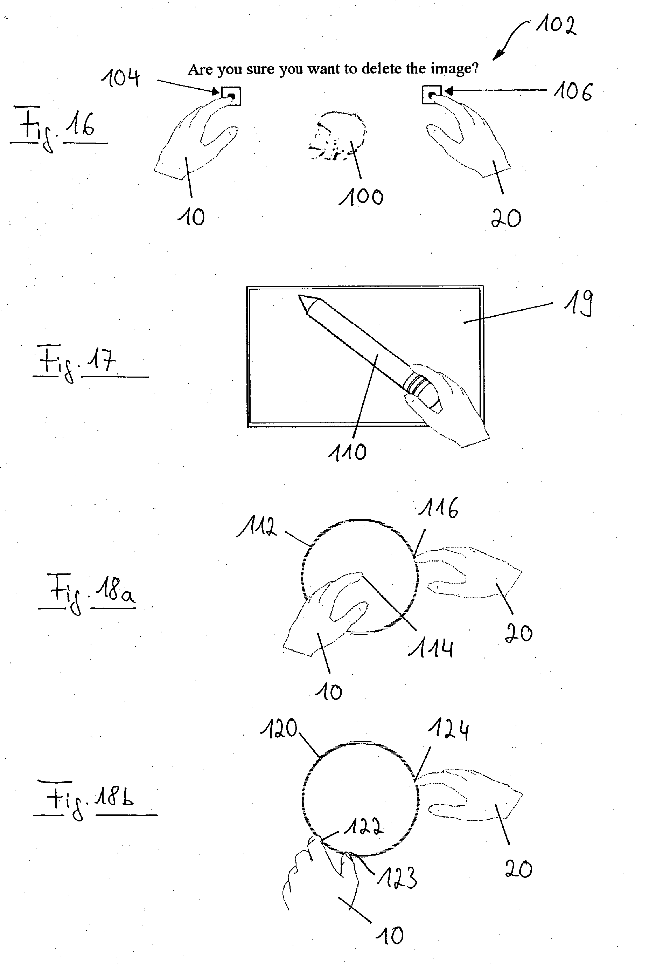

Weil beim versehentlichen Löschen mancher Bilder Informationen verloren gehen können, kann eine erfindungsgemäß ausgestaltete Applikation auch hier mehr Sicherheit bieten. Beispielsweise zeigt die Figur 16, wie man eine Löschbestätigung für das Bild 100 anfordert und erfindungsgemäß auslöst, nämlich durch eine zweihändige Berührung der Schaltflächen 104 und 106 nach einer Anfrage 102. Die Figur 17 zeigt einen Anwendungsfall, bei dem ein reales Objekt vermessen werden kann, beispielsweise das Zeigegerät 110, das an dem Bildschirmabschnitt 19 gebracht wird. Wenn ein entsprechender Modus eingestellt wird, oder das Objekt 110 für längere Zeit auf dem Bildschirm verbleibt, kann dadurch ausgelöst werden, dass die Berührungsfläche vermessen wird, bzw. die Anzahl der Berührungen gezählt wird und entsprechende Objektabmessungen sind erfassbar.

Die Figuren 18a und 18b zeigen, wie mit Hilfe entsprechender Gesten ein geometrisches Objekt, hier ein Kreis am Bildschirm erzeugt werden kann. In Figur 18a wird der Kreis 112 dadurch erzeugt, dass eine Fingerspitze auf den Mittelpunkt 114 und eine Fingerspitze auf einen Umfangspunkt 116 deutet, während in Figur 18b ein Kreis 120 durch drei Umfangspunkte 122, 123 und 124 eingegeben wird.

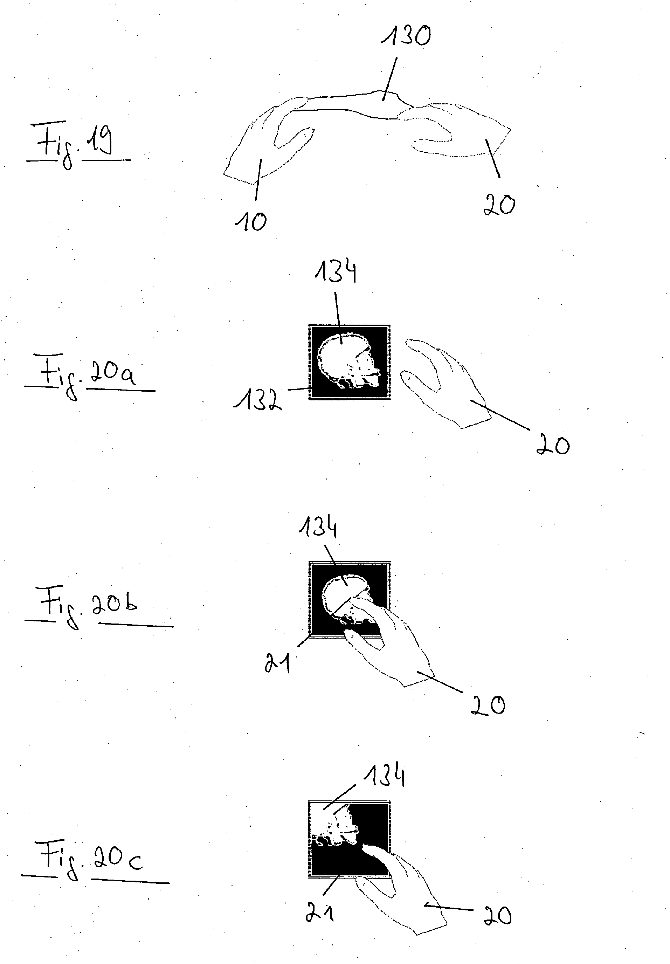

Auch medizinische Implantate können in ihrer Darstellung auf dem Bildschirm manipuliert werden, wie beispielsweise die Figur 19 schematisch zeigt. Das Implantat 130 kann zum Beispiel durch Vergrößerungs-, Verkleinerungs- oder Dreh-Gesten, wie sie oben schon beschrieben worden sind, verändert werden. Wenn auf dem Bildschirm andere Bilddaten zur Verfügung stehen, z.B. Anatomie-Strukturen, in welches das Implantat eingebracht werden kann, kann schon die geeignete Implantatgröße vorab am Bildschirm geplant werden. Es ist dann auch möglich, den Computer das angepasste Implantat mit verschiedenen hinterlegten und zur Verfügung stehenden Implantatgrößen vergleichen zu lassen. Wenn ein geeignetes Implantat schon zur Verfügung steht und die Datenbank entsprechendes ausgibt, kann genau dieses Implantat gewählt oder bestellt werden, oder aber notwendige Anpassungen des nächst größeren Implantats werden schon berechnet und ausgegeben.

Anhand der Figuren 20a bis 20c wird aufgezeigt, wie gemäß der Erfindung eine Geste unterschiedlich und abhängig von dem Teil des Bildes interpretiert werden kann, auf den die Geste angewendet wird. Das dargestellte Bild weist einen hellen Kopfumriss 134 und einen dunklen Bereich 132 auf, und wenn mit dem Finger auf den hellen Bereich gezeigt wird, kann diese Geste beispielsweise als ein Befehl zum Scrollen durch verschiedene Schnittebenen aufgefasst werden, wenn der Finger über den hellen Bereich gezogen wird (Figur 20b), z.B. wenn der Finger mit seiner Spitze 21 aber auf dem dunklen Bereich aufgesetzt wird, wird diese Geste als ein Befehl zum Verschieben des Bildes interpretiert, wie dies in Figur 20c dargestellt ist.

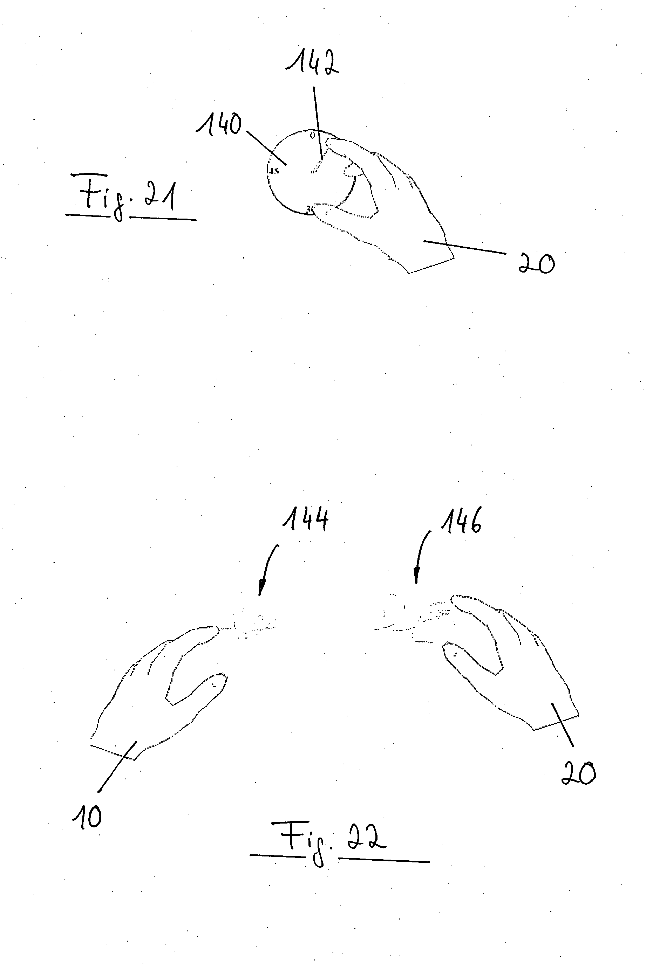

Manchmal müssen in Operationssälen gewisse Zeiten eingehalten werden, speziell gewisse Wartezeiten, wenn beispielsweise ein Material aushärten muss. Um diese Wartezeiten messen zu können, kann in einem geeigneten Modus die Gestenerkennung zur Darstellung und zum Einstellen einer Uhr bzw. eines Countdowns genutzt werden, und in Figur 21 ist dargestellt, dass in diesem Modus beispielsweise eine Berührung mit zwei Fingern eine Countdown-Uhr 140 auf dem Monitor erscheinen lässt. Wird dann der Zeigefinger um den Daumen herum gedreht, führt dies zu einer Verschiebung des Zeigers 142, und der Countdown kann mit dieser voreingestellten Zeit beginnen.

Eine weitere Anwendung ist eine Signatur, die durch Mehrfachberührung des Bildschirms eingegeben wird. Wenn gleichzeitig oder aufeinander folgend mit zwei Händen und durch entsprechende Gesten eine Linienfolge eingegeben wird, kann dies zu einer sehr eindeutigen Identifizierung des Verwenders genutzt werden.

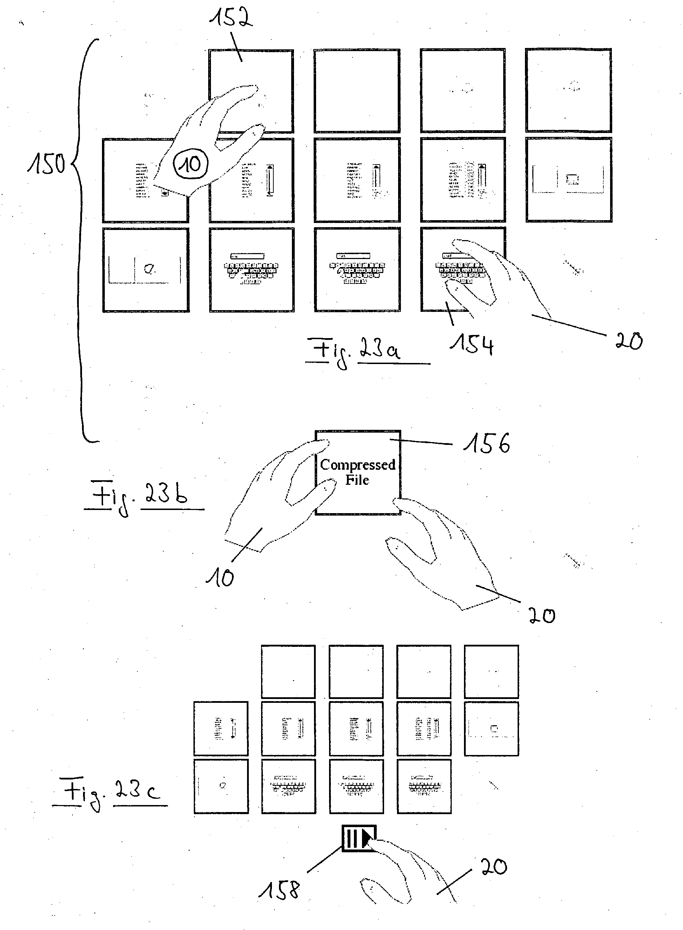

Die Ausführungsform, die anhand der Figuren 23a bis 23c erläutert werden soll, betrifft eine Mehrfachauswahl von Bildelementen bzw. Bildobjekten oder den Umgang mit solchen Elementen oder Objekten. In der Figur 23a ist eine Anzahl von Bildobjekten, nämlich kleineren Bildern 150 dargestellt, und mit Hilfe von zwei Berührungen wird in einem entsprechenden Auswahlmodus das erste Bild 152 und das letzte Bild 154 einer auszuwählenden Bildfolge angewählt. Die erste Berührung mit der Hand 10 beim Bild 152 bleibt dabei solange aktiv bis das Bild 154 ebenfalls angewählt worden ist. Die Vielzahl von Bildern kann nun unterschiedlichen Bearbeitungen zugeführt oder in unterschiedlicher Weise verwendet werden. Eine Verwendung ist in Figur 23b dargestellt. Wenn nämlich vom Zustand der Figur 23a ausgehend eine Verkleinerungs- bzw. Zoom-In-Geste mit den beiden Händen gemacht wird, wobei beispielsweise die beiden Fingerspitzen aufeinander zu geführt werden, während sie den Bildschirm berühren, kann eine komprimierte Datei erzeugt werden, die alle ausgewählten einzelnen Darstellungen umfasst und die in Figur 23b mit dem Bezugszeichen 156 bezeichnet ist. Eine weitere, in Figur 23c dargestellte Anwendung ist beispielsweise das Abspielen eines Films oder einer Bildfolge aus ausgewählten Dateien, wobei dies ebenfalls durch eine entsprechende Geste oder durch das Aktivieren eines Abspiel-Knopfes geschehen kann.

## Patentansprüche

1. Verfahren zur Anzeige und/oder Bearbeitung bzw. Verarbeitung von Bilddaten medizinischen oder medizintechnischen Ursprungs, unter Verwendung eines Anzeigegeräts (1) mit mindestens einem Bildschirm (2, 3), wobei
- die Bilddaten durch eine im Anzeigegerät (1) integrierte Computer-Datenverarbeitungseinheit (4) verarbeitet werden, um Bildausgaben (14) zu erzeugen und/oder die Bilddaten zu ändern bzw. zu bestätigen,
- die Bilddaten durch Eingaben am Bildschirm (2, 3) selbst manipuliert, erzeugt oder aufgerufen werden, und wobei
- die Eingaben mit Hilfe der Datenverarbeitungseinheit (4) über eine Gestenerkennung identifiziert werden, wobei die Gesten per Hand oder mit Gestenerzeugungsgeräten erzeugt werden.

2. Verfahren nach Anspruch 1, bei dem die Eingaben Steuerungseingaben für die Bilddaten-Darstellung am Bildschirm (2, 3) sind.

3. Verfahren nach Anspruch 1 oder 2, bei dem die Eingaben an einem berührungsempfindlichen Bildschirm (2, 3) oder einem Bildschirm (2, 3) erfolgen, der Präsenzen in der Nähe seiner Oberfläche feststellen kann.

4. Verfahren nach einem der Ansprüche 1 bis 3, bei dem der Bildschirm (2, 3) mehrere gleichzeitige Berührungen der Bildschirmoberfläche oder Präsenzen an der Bildschirmoberfläche identifizieren kann.

5. Verfahren nach einem der Ansprüche 1 bis 4, bei dem bei der Gestenerkennung eine definierte Abfolge mehrerer Eingaben, gleichzeitige Eingaben an mehreren Bildschirmstellen oder einzelne Eingaben über einen gewissen Zeitraum als Eingabegesten identifiziert werden.

6. Verfahren nach einem der Ansprüche 1 bis 5, bei dem die Eingabegesten punkt- oder flächenförmige Berührungen der Bildschirmoberfläche oder Präsenzen an der Bildschirmoberfläche sind, wobei insbesondere zwischen diesen Eingaben unterschieden wird.

7. Verfahren nach einem der Ansprüche 1 bis 6, bei dem die Bildausgabe und/oder die Eingabe auf mindestens zwei nebeneinander angeordneten Bildschirmen (2, 3) erfolgt, wobei insbesondere einer dem Aufrufen bzw. der Auswahl von Bilddaten bzw. Bilddatensätzen und ein anderer der Manipulation oder Erzeugung von Bilddaten bzw. Bilddatensätzen dient.

8. Verfahren nach einem der Ansprüche 1 bis 7, bei dem Eingaben durch flächenförmige Berührungen der Bildschirmoberfläche oder Präsenzen an der Bildschirmoberfläche als andere Eingabebefehle interpretiert werden als Eingaben durch punktförmige Berührungen der Bildschirmoberfläche oder Präsenzen an der Bildschirmoberfläche, insbesondere bei Eingaben an demselben Eingabefeld.

9. Verfahren nach einem der Ansprüche 1 bis 8, bei dem Eingabegesten, insbesondere Eingaben mit mehreren oder flächenförmigen Berührungen der Bildschirmoberfläche oder Präsenzen an der Bildschirmoberfläche zur Steuerung von Bildeigenschaften, insbesondere des Zoomfaktors, der Helligkeit, des Kontrastes oder von Auswahlfeldern (21) verwendet werden.

10. Verfahren nach einem der Ansprüche 1 bis 9, bei dem Eingabegesten, insbesondere Eingaben mit mehreren oder flächenförmigen Berührungen der Bildschirmoberfläche oder Präsenzen an der Bildschirmoberfläche zur Vergrößerung eines Bildbereichs (27) oder Bildschirmbereichs verwendet werden.

11. Verfahren nach einem der Ansprüche 1 bis 10, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben zur Erzeugung eines Polygonzugs (35), insbesondere zur Abgrenzung bzw. Definition von Bildbereichen verwendet werden.

12. Verfahren nach einem der Ansprüche 1 bis 11, bei dem Eingabegesten, insbesondere linienförmige Eingabegesten, speziell mehrere gleichzeitig erfolgende lineare Eingabegesten zum Spiegeln eines Bildes verwendet werden.

13. Verfahren nach einem der Ansprüche 1 bis 12, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben zum Aufrufen eines versteckten Eingabefelds (45) und zur Auswahl eines Eingabebefehls (46) in dem Feld verwendet werden.

14. Verfahren nach einem der Ansprüche 1 bis 13, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben zum Aufrufen und/oder Bedienen einer Bildschirmtastatur (52) verwendet werden, wobei insbesondere Spezialtasten (54) aufgerufen und betätigt werden können.

15. Verfahren nach einem der Ansprüche 1 bis 14, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben zur Betätigung von Scrollleisten (61) mit unterschiedlichen Scrollgeschwindigkeiten oder Auswahllistenkriterien je nach Eingabetyp verwendet werden.

16. Verfahren nach einem der Ansprüche 1 bis 15, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben zur Auswahl eines Punktes oder Bereichs in einem Linien- oder Flächendiagramm verwendet werden, wobei daraufhin insbesondere:
- die Koordinaten des Punktes (72) oder der Fläche an den Diagrammachsen (74, 76) oder an einer zugeordneten Bildstelle ausgegeben werden;
- die Skalierung des Diagramms durch abfolgende weitere Gesten geändert wird;
- Diagrammteile vergrößert, verkleinert oder verschoben werden;
wobei diese Manipulationen einzeln oder in jedweder Kombination durchgeführt werden können.

17. Verfahren nach einem der Ansprüche 1 bis 16, bei dem eine Eingabegeste, bei der eine Hand (20) flach auf bzw. an einen Bildschirmbereich (17) gebracht wird, dazu verwendet wird, durch die Identifizierung der entstehenden mehreren oder flächenförmigen Berührungen der Bildschirmoberfläche oder Präsenzen an der Bildschirmoberfläche die Hand (20) als rechte oder linke Hand zu bestimmen und dann insbesondere in einem Datensatz zu hinterlegen, ob der Benutzer Rechts- oder Linkshänder ist.

18. Verfahren nach einem der Ansprüche 1 bis 17, bei dem eine Eingabegeste mit zwei punktförmigen Eingabe, insbesondere zwei Fingerspitzen (21, 22), zur Eingabe einer Linie (80), speziell einer bemaßten Linie (82), in das Bild bzw. den Bilddatensatz verwendet wird, wobei insbesondere der Abstand der punktförmigen Eingaben die Länge der Linie bestimmt und/oder verändert.

19. Verfahren nach einem der Ansprüche 1 bis 18, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben verwendet werden, um zwei- oder dreidimensionale Darstellungen eines Bilddatensatzes, insbesondere eines Patientendatensatzes, der mit einem medizintechnischen Bildgebungsverfahren erstellt worden ist, zu manipulieren, insbesondere um
- die Darstellungen (84) zu drehen, zu kippen oder zu spiegeln;
- eine Schnittebene (88) im dargestellten Bild (84) zu bestimmen oder zu verändern und/oder entsprechend eine Schnittdarstellung (86) anzuzeigen;
- die Darstellung (84) zu verschieben;
wobei eine oder mehrere der obigen Manipulationen durchgeführt werden können.

20. Verfahren nach einem der Ansprüche 1 bis 19, bei dem Eingabegesten, insbesondere gleichzeitige bzw. aufeinanderfolgende punktförmige Eingaben verwendet werden um Bildpunkte (90, 96) paarweise oder mehrfach zuzuordnen, insbesondere dieselben Bildpunkte in verschiedenen Ansichten eines Bilddatensatzes.

21. Verfahren nach einem der Ansprüche 1 bis 20, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben dazu verwendet werden, Befehlsbestätigungen zu tätigen.

22. Verfahren nach einem der Ansprüche 1 bis 21, bei dem eine Eingabegeste, bei der ein Objekt (110) auf oder an den Bildschirm gebracht wird, als Befehl zur Vermessung des Objekts (110) identifiziert wird, insbesondere wenn das Objekt über einen definierten Zeitraum auf oder an dem Bildschirm belassen wird.

23. Verfahren nach einem der Ansprüche 1 bis 22, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben dazu verwendet werden, geometrische Figuren oder Körper als Konturen zu erzeugen, insbesondere Kreise (112, 120) oder Rechtecke.

24. Verfahren nach einem der Ansprüche 1 bis 23, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben dazu verwendet werden, Objekte (130) zu skalieren bzw. in der Größe anzupassen, speziell Implantate.

25. Verfahren nach einem der Ansprüche 1 bis 24, bei dem der Bildinhalt eines von einer Eingabegeste betroffenen Bildbereiches (132, 134), insbesondere seine Helligkeit, bestimmt wird, wobei der Eingabegeste je nach dem Bildinhalt eine spezielle und bei unterschiedlichen Inhalten verschiedene Steuerungsfunktion zugewiesen wird.

26. Verfahren nach einem der Ansprüche 1 bis 25, bei dem Eingabegesten, insbesondere gleichzeitige bzw. aufeinanderfolgende punktförmige Eingaben dazu verwendet werden, eine Uhr bzw. einen Countdownzähler (140) auf dem Bildschirm zu aktivieren und/oder einzustellen und auszulösen.

27. Verfahren nach einem der Ansprüche 1 bis 26, bei dem Eingabegesten, insbesondere gleichzeitige bzw. aufeinanderfolgende punktförmige Eingaben dazu verwendet werden, eine Signatur (144, 146) über den Bildschirm einzugeben.

28. Verfahren nach einem der Ansprüche 1 bis 27, bei dem Eingabegesten, insbesondere Eingaben mit mehreren oder flächenförmigen Berührungen der Bildschirmoberfläche oder Präsenzen an der Bildschirmoberfläche oder gleichzeitige bzw. aufeinanderfolgende punktförmige Eingaben dazu verwendet werden, eine Mehrfachauswahl von Bildelementen zu treffen, insbesondere von aneinandergereihten Bildelementen (152-154) durch Anwahl des ersten und letzten Bildelements.

29. Verfahren nach Anspruch 28, bei dem, wenn die Bildelemente Dateien repräsentieren, durch eine zusammenlaufende Geste eine komprimierte Datei aus den Dateien erstellt wird, oder, wenn die Bildelemente Bilder sind, durch eine Geste oder die Berührung eines Bildschirmschalters eine Bildabfolge aus den ausgewählten Bildelementen gestartet wird.

30. Programm, das, wenn es auf einem Computer läuft oder in einem Computer geladen ist, den Computer veranlasst, ein Verfahren gemäß einem der Ansprüche 1 bis 29 durchzuführen.

31. Computerprogramm-Speichermedium, das ein Programm nach Anspruch 30 aufweist.
